(19) Europäisches Patentamt
European Patent Office
Office européen des brevets

(11) EP 1 997 382 B1

(12) # EUROPÄISCHE PATENTSCHRIFT

(45) Veröffentlichungstag und Bekanntmachung des Hinweises auf die Patenterteilung:
**20.09.2017 Patentblatt 2017/38**

(21) Anmeldenummer: **08007825.6**

(22) Anmeldetag: **23.04.2008**

(51) Int Cl.:
***A01N 47/16*** *(2006.01)*
***A01N 37/22*** *(2006.01)*
***A01N 65/00*** *(2009.01)*
***A01N 25/30*** *(2006.01)*
***A01P 17/00*** *(2006.01)*
***A61K 8/49*** *(2006.01)*
***A61K 8/44*** *(2006.01)*
***A61K 8/81*** *(2006.01)*
***A61K 8/92*** *(2006.01)*
***A01N 37/46*** *(2006.01)*
***A01N 43/16*** *(2006.01)*
***A01N 25/04*** *(2006.01)*
***A61Q 17/02*** *(2006.01)*
***A61K 8/97*** *(2017.01)*
***A61K 8/42*** *(2006.01)*
***A61K 8/60*** *(2006.01)*
***A61K 8/37*** *(2006.01)*

(54) **Insektenschutzmittel auf Emulsionsbasis**

Insect repellent based on emulsion

Produit de protection contre les insectes à base d'émulsion

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**
Benannte Erstreckungsstaaten:
**AL BA MK RS**

(30) Priorität: **31.05.2007 DE 102007026052**

(43) Veröffentlichungstag der Anmeldung:
**03.12.2008 Patentblatt 2008/49**

(73) Patentinhaber: **Beiersdorf AG**
**20245 Hamburg (DE)**

(72) Erfinder:
- **von der Fecht, Stephanie**
  **22880 Wedel (DE)**
- **Schulz, Jens, Dr.**
  **25462 Rellingen (DE)**
- **Kröpke, Rainer**
  **22869 Schenefeld (DE)**
- **Nielsen, Jens**
  **24558 Henstedt-Ulzburg (DE)**
- **Heptner, Astrid**
  **22527 Hamburg (DE)**

(74) Vertreter: **Hartmann, Jost**
**Beiersdorf AG**
**Unnastrasse 48**
**20253 Hamburg (DE)**

(56) Entgegenhaltungen:
**EP-A1- 1 738 745** **EP-A1- 1 745 722**
**DE-T2- 69 005 801**

- **DISCLOSED ANONYMOUSLY: "Neue emulgatorenken für kosmetische zubereitungen von sensorisch eleganten hautpflegeprodukten", RESEARCH DISCLOSURE, MASON PUBLICATIONS, HAMPSHIRE, GB, Bd. 510, Nr. 47, 1. Oktober 2006 (2006-10-01), Seite 1326, XP007136726, ISSN: 0374-4353**
- **ANONYMOUS: "Neue Emulgatoren für kosmetische Zubereitungen für die Hautpflege", RESEARCH DISCLOSURE, MASON PUBLICATIONS, HAMPSHIRE, GB, Bd. 491, Nr. 32, 1. März 2005 (2005-03-01) , XP007134839, ISSN: 0374-4353**
- **ANONYMOUS: "Neue Emulgatoren für kosmetische AP/Deo Zubereitungen", RESEARCH DISCLOSURE, Bd. 510, Nr. 47, 1. Oktober 2006 (2006-10-01), XP55029125, ISSN: 0374-4353**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 997 382 B1

## Beschreibung

Die vorliegende Erfindung betrifft kosmetische Formulierungen auf Emulsionsbasis zur Insektenabwehr umfassend a.) einen oder mehrere Insektenrepellentwirkstoffe und b.) Natriumstearoylglutamat und/oder Sucrosepolystearat sowie c.) hydriertes Polyisobutylen. Die Kombination ermöglicht eine Wirksamkeitsteigerung in der Insektenabwehr um bis zu 30%. Insektenschutzmittel (Insektenvertreibungsmittel, Insektenabwehrmittel, Repellentien, Repellents) sind Präparate, die zur Abwehr und/oder Vertreibung von Insekten, aber auch Zecken und Milben äußerlich angewendet werden und verhindern sollen, dass diese auf der Haut aktiv werden. Insektenabwehrmittel sollen die Haut vor Belästigung durch Blut saugende oder beißende Insekten und andere Parasiten und/oder Lästlinge schützen, indem sie diese abwehren, bevor sie sich auf der Haut niederlassen. Die Mittel wirken dementsprechend nicht als Kontaktgifte, sondern nur als Abwehrmittel, da sie die Tiere nicht töten, sondern lediglich vertreiben.

Als Repellent-Wirkstoff ist beispielsweise 1-Piperidincarboxylsäure 2-(2-hydroxyethyl)-1-methylpropylester (INN: Icaridin, CAS-Nummer: 119515-38-7, Elincs-Nummer: 423-210-8) bekannt, der die folgende Struktur aufweist:

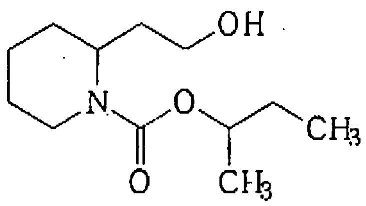

Ein weiterer eingesetzter Repellent-Wirkstoff ist der 3-(N-n-Butyl-N-acetyl-amino)propionsäureethylester (auch als Ethylbutylacetylaminopropionat oder Repellent 3535 bezeichnet), welcher sich durch die folgende Strukturformel auszeichnet:

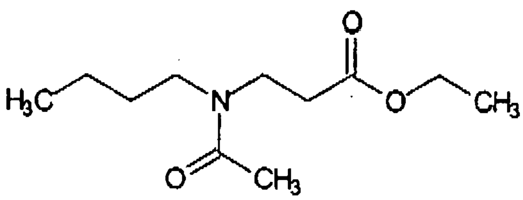

Nicht zuletzt kennt der Fachmann den Repellent-Wirkstoff N,N-Diethyl-3-methylbenzamid (Handelsbezeichnung: Meta-delphene, DEET). Diese Stoffe sind als Repellentwirkstoffe gegen Insekten, insbesondere Mücken oder Zecken, vielfach beschrieben, wie z.B. in DE 2246433, DE 19645250, DE 19645920.

In der WO 2006096876 und WO 2005034626 werden Dihydronepetalactone als Repellentien gegen Insekten offenbart.

Katzenminze oder auch Nepeta ist als Mückenabwehrmittel bekannt und soll eine etwa zehn Mal stärkere Abwehr gegen Mücken als herkömmliche Abwehrmittel aufweisen.

In der DE 102005033845 werden des weiteren u.a bestimmte Parfuminhaltsstoffe zur Abwehr von Mücken, Sandfliegen, Läusen, Bremsen, Wespen, Bienen, Fliegen und Zecken erwähnt.

Ein Problem ist dabei, dass ein Repellentwirkstoff, der beispielsweise gegen Mücken abwehrend wirkt, diese Abwehrwirkung nicht oder nur eingeschränkt gegen andere Insekten aufweist.

Um wirksam Insektenabwehrend zu wirken müssen die Repellentien zu einem relativ hohen Anteil zugesetzt werden. Um einen Vollschutz gegen Zecken oder auch der sehr aggressiven Anopheles Mücke zu erzielen, muss ein Zusatz von bis 20% des Repellents zugesetzt werden. Problem dabei ist, dass Zubereitungen mit einer hohen Konzentration an Repellentien bei Menschen mit besonders empfindlicher Haut in Einzelfällen zu Hautirritationen führen können. Ferner haben die Repellent-Wirkstoffe einen auch für die menschliche Nase wahrnehmbaren und auf Mitmenschen nicht gerade anziehend wirkenden Eigengeruch, der die Anwendung von Insektenabwehrmitteln nicht gerade attraktiv macht. Die abweisende Wirkung der Zubereitung erstreckt sich also nicht allein auf Insekten sondern darüber hinaus unter Umständen auch auf Mitmenschen, wenn diese über einen ausgeprägten Geruchsinn verfügen.

Die Zubereitungen des Standes der Technik haben bei derartig hohen Einsatzkonzentrationen den Nachteil, insbesondere bei Menschen mit empfindlicher oder zu Allergien neigender Haut nicht besonders verträglich zu sein und in Einzelfällen zu Hautreizungen zu führen.

Wünschenswert ist es eine Zubereitung zur Verfügung zu stellen, die einerseits insektenabwehrend wirkt und andererseits hautverträglich gestaltet ist und vorteilhaft einen für die menschliche Nase angenehm wohlriechenden Duft

verströmt.

Weiterer Nachteil der aus dem Stand der Technik bekannten Insektenrepellentien auf Emulsionsbasis ist, dass sie in Abhängigkeit vom Emulgator nach ca. 2 - 4 Stunden ihre Repellentien wirksamkeit veringern oder ganz verlieren.

So offenbart die EP 1738745 A1 W/S- und W/O-Emulsionen umfassend Polyisobuten und einem Repellentwirkstoff Icaridin.

Um dann dennoch einen besseren Schutz zu erreichen benötigt man wiederum Zusatzstoffe, wie Filmbildner, wie Gerandiol, die dann aber die Langzeitstabilität der Zubereitung einschränken.

Es war daher die Aufgabe der vorliegenden Erfindung, kosmetische Zubereitungen auf Emulsionsbasis mit einem Gehalt an Insektenrepellentieri zur Verfügung zu stellen, die eine derartige Wirksamkeitsverminderung und/oder Langzeitinstabilität nicht aufweisen.

Die Erfindung beschreibt eine Zubereitung auf W/O- oder W/S-Emulsionsbasis umfassend a.) einen oder mehrere Insektenrepellentwirkstoffe und b.) Natriumstearoylglutamat und/oder Sucrosepolystearat sowie c.) hydriertes Polyisobutylen. Als Insektenrepellentien können bevorzugt gewählt werden 1-Piperidincarboxylsäure 2-(2-hydroxyethyl)-1-methylpropylester (Icaridin), 3-(N-n-Butyl-N-acetyl-amino)propionsäureethylester (IR 3535), N,N-Diethyl-3-methylbenzamid (DEET), Ethylbutylacetylaminopropionat (EBAAP), Dihydronepetalactone (DHPL) und/oder Extrakte der Katzenminze.

Bevorzugt wird 3-(N-n-Butyl-N-acetyl-annino)propionsäureethylester als Repellent gewählt, insbesondere zu einem Anteil von bis zu 25 Gew.%, bevorzugt von etwa 15 Gew.%, bezogen auf die Gesamtmasse der Zubereitung.

Dihydronepetalactane sind beispielweise in der WO 2006096876 und WO 2005034626 beschrieben. Die Katzenminzen (Nepeta) bezeichnen eine Pflanzengattung der Familie der Lippenblütler (Lamiaceae). Erfindungsgemäß werden als Extrakte alle möglichen Extrakte, Lösungen oder Dispersionen der Nepetapflanze oder deren Teile verstanden.

Bei den in den Nepeta enthaltenen ätherischen Ölen handelt es sich in den Hauptbestandteilen um Citral, Citronellol, Geraniol und Limonen, sowie Nepetalacton, Gerb- und Bitterstoffe. Der Wirkstoff, der die Pflanze für Katzen so unwiderstehlich macht, ist Actinidin, ein iridoides Glykosid, das dem vergleichbaren Wirkstoff des Baldrian sehr ähnlich ist (BOWN, 1995). Die Nepeta enthält etwa 0,2-0,7 % ätherische Öle.

Der ölige Extrakt der Katzenminze, insbesondere aufgereinigt, ist wie jedes andere Naturöl sehr schlecht spreitend und sehr polar. Es ist deshalb unkosmetisch und verteilt den herkömmlichen kosmetischen Formeln eine ölig, fettige Phase. Es wird als schwer verteilbar wahrgenommen. Der polare Charakter des Extraktes führt zudem sehr schnell zu Instabilitäten, wie z.B, Öl- oder Wasserabscheidung.

Erfindungsgemäß ist diese Schwierigkeit der kosmetischen Formulierung durch die Kombination mit den Natriumstearoylglutamat, Sucrosepolytsearat und/oder hydriertes Polyisobutylen überwunden worden.

Die erfindungsgemäße Zubereitung kann die Repellentien, bevorzugt IR 3535, in unterschiedlichen Formen enthalten.

Es ist erfindungsgemäß vorteilhaft, wenn die Repellentien in einer Gesamtkonzentration von 0,1 bis 40 Gew.% und erfindungsgemäß bevorzugt, wenn die Repellentien in einer Gesamtkonzentration von 10 bis 20 Gew.%, jeweils bezogen auf das Gesamtgewicht der Zubereitung, in dieser enthalten sind.

Die erfindungsgemäße Zubereitung umfasst Natriumstearoylglutamat und/oder Sucrosepolytsearat sowie hydriertes Polyisobutylen, bevorzugt alle drei Substanzen. Natriumstearoylglutamat ist beispielsweise als Eumulgin ® SG (Emulgade SG) der Firma Carechemicals erhältlich, eine Mischung aus Sucrosepolytsearat und hydriertem Polyisobutylen ist als Emulgade® Sucro der Firma Carechemicals zu beziehen.

In EP 766959 B1, EP 928608 B1 und EP 1151743 A1 werden diese Emulgatoren ebenfalls beschrieben.

Die Kombination von Natriumstearoylglutamat und/oder Sucrosepolytsearat sowie hydriertes Polyisobutylen mit den Insektenrepellentien in Emulsionen führt dazu, dass die Wirksamkeit, d.h. die Repellentienverfügbarkeit, um bis zu 30% gesteigert werden kann. Bevorzugt umfasst die Zubereitung auf Emulsionsbasis mindestens zwei der drei Substanzen, bevorzugt Natriumstearoylglutamat und Sucrosepolystearat insbesondere alle drei.

Natriumstearoylglutamat und Sucrosepolystearat werden bevorzugt im Verhältnis 1:10 bis 10:1, insbesondere 1:5 bis 5:1 eingesetzt.

In einem Käfigtest wurde die Schutzwirkung der Zubereitungen gegenüber Stechmücken untersucht. Basis für die Tests war der Entwurf einer Richtlinie der amerikanischen *Environmental Protection Agency* (EPA, 2000). Die in der vorliegenden Untersuchung verwendeten Testkäfige sind eine Weiterentwicklung der in dieser Richtlinie erwähnten Käfige. Sie werden mit klimatisierter Luft belüftet, die Testarme werden von außen an ein Fenster definierter Größe gehalten.

Für jede Repellentprobe wurde der Zeitpunkt des Erst- und Zweitstiches sowie die zeitliche Dauer bis zum Erreichen eines 95%igen Schutzes vor Stichen ermittelt. Die Repellentproben wurden von 5 Probanden (3 weibliche und 2 männliche, Alter zwischen 21 und 34 Jahren) über eine maximale Dauer von 8 Stunden alle 30 Minuten an einer stechfreudigen Käfig population der Geibfiebermücke *Aedes aegypti* getestet.

Die Repellentproben wurden auf eine definierte Hautfläche auf einem Unterarm der Probanden aufgetragen,

die Auftragsmenge entsprach den Vorgaben der Richtlinie: pro 600 cm2 Haut 1,5 g Lotion oder 1 g Flüssigspray. Direkt nach dem Auftragen wurde zum ersten Mal getestet. Die behandelte Hautfläche war größer als das Testfenster im Boden des Testkäfigs, um sicher zu gehen, dass die gesamte exponierte Haut mit Wirkstoff versetzt war.

Der andere, unbehandelte Unterarm der Probanden diente als Nullkontrolle. Diese wurde vor jedem Einzeltest durchgeführt und durfte einen Wert von 10 Stichen pro 30 Sekunden nicht unterschreiten, Die genaue Zeit bis zum 10. Stich wurde notiert, mit Hilfe dieses Zeitwertes wurde die prozentuale Schutzwirkung gemäß folgender Formel berechnet:

$$\%R= (SKxY-ST)xIOO-(SKxY)$$

| | | | |
|---|---|---|---|
| ***%R*:** | Prozentuale | | Repellentwirkung |
| ***SK*:** | Anzahl | Stiche | Kontrollarm |
| ***ST*:** | Anzahl | Stiche | Testarm |

**Y:** Dauer der Exposition des Testarms (2 Mm) + Dauer der Exposition des Kontrollarms (max. 0,5 Mm)

Jeder Proband testete pro Versuchstag eine zuvor ausgeloste Repellentprobe an einem Käfig. Zwischen den Tests und der Nullkontrolle lag eine Belüftungszeit von 5 Minuten, um eine Anreicherung von Lock- oder Wirkstoffen im Käfig zu vermeiden. Dazu wurden die Käfige über ein spezielles Schlauchsystem mit konstant warmer und feuchter Luft durchspült (26 ± 1°C, 70 ± 10%). Während der Tests wurde das Schlauchsystem nicht angeschlossen, da die einströmende Luft die Testmücken bei der Orientierung in Richtung des Testarmes behindert, außerhalb der Tests wurden die Testkäfige allerdings dauerhaft belüftet. Vor Versuchsbeginn wurde die Aktivität der Testmücken mit dem unbehandelten Unterarm des Versuchsleiter geprüft, unterschritt die Stichzahl einen Wert von mindestens 10 Stichen innerhalb von 30 Sekunden wurden 30 neue Testmücken eingesetzt. Während der Tests wurden blutsaugende Testmücken im Anschluss ersetzt, um zu gewährleisten, dass sich stets eine konstante Anzahl an stechfreudigen Testmücken im Testkäfig befindet. Die Abbildung 1 zeigt die Ergebnisse des Käfigtests. Die Zubereitungen enthielten 15 Gew.% IR 3535 als Repellentwirkstoff sowie Natriumstearoylglutamat, Sucrosepolystearat und hydriertes Polyisobutylen (s. Abbildung 1).

Die Zunahme an Insektenabwehrwirksamkeit zeigt sich insbesondere bei der Kombination aller drei Substanzen mit IR 3535 (rechten 2 Balken in Abb.1).

Die Verwendung der Kombination aus Insektenrepellentwirkstoffe, insbesondere 3-(N-n-Butyl-N-acetyl-amino)propionsäureethylester, und Natriumstearoylglutamat, Sucrosepolystearat und/oder hydriertes Polyisobutylen zur Steigerung der Insektenabwehrwirkung um bis zu 30% gegenüber einer Zubereitung ohne Natriumstearoylglutamat, Sucrosepolytsearat und/oder hydriertes Polyisobutylen ist damit bevorzugt.

Neben der Insektenabwehr sind aber vor allem die Hautverträglichkeit und das Hautgefühl entscheidende Eigenschaften eines topisch zu applizierenden insbesondere kosmetischen Produktes.

Es nützt eine hervorragende Repellentwirksamkeit nichts, wenn das Kosmetikprodukt zu Unverträglichkeiten führt.

Eine der häufig auftretenden Hautunverträglichkeiten ist der sog. Stinging-Effekt, der sich insbesondere im Gesicht unangenehm bemerkbar macht. Der Stinging-Effekt tritt insbesondere im Nasolabial-Bereich auf und bedeutet ein subjektives Missempfinden der Probanden.

Um solche unerwünschten Effekte für Kosmetika möglichst auszuschliessen werden vor Produkteinführung sog. Stinging-Tests durchgeführt. Jeweils zwei Produkte werden dabei im dirketen Paarvergleich auf ihre Hautverträglichkeit getestet. Als interner High-Standard (Benchmark) wird dabei ein als hautverträglich bekanntes handelsübliches Produkt verglichen (NIVEA Visage Day Cream normal mixed).

In zahlreichen Untersuchungen wurde nun festgestellt, dass ein wesentlicher Beitrag zum Stinging durch die in vielen Kosmetika enthaltenen Parabene hervorgerufen wird, insbesondere wenn diese mit einem Anteil von mehr als 0,3 Gew.% in der Rezeptur vorliegen.

Parabene werden als Konservierungsmittel eingesetzt, so dass deren Ersatz mitunter schwierig ist.

Es wurde nun überaschenderweise fest gestellt, dass die erfindungsgemäßen Zubereitungen sehr gute Repellentwirksamkeiten zeigen aber einen Stinging-Effekt nicht aufweisen.

Die erfindungsgemäßen Zubereitungen lassen sich daher bevorzugt mit einem Anteil von bis zu 0,3 Gew.% Konservierungsmittel, wie insbesondere Parabene, herstellen.

Bevorzugt kann auf den Zusatz von Konservierungsmitteln, wie z.B. Parabenen, ganz verzichtet werden.

D.h es ist erstmals möglich Repellenthaltige Zubereitungen zur Verfügung zu stellen, die gleichzeitig verbesserte Verträglichkeit aufweisen und insbesondere keine Stinging-Effekte aufweisen.

Die erfindungsgemäßen Zubereitungen sind daher bevorzugt frei von bakteriziden-, fungiziden-, sporicidally d.h. sporenabtötenden, wirksamen Mitteln und/oder frei von Konservierungsmitteln. Weiterhin sind sie bevorzugt frei

von Formaldehyd, Formaldehyddonatoren wie Diazolidinyl urea, Imidazolidinyl urea und/oder DMDM Hydantoin sowie insbesondere frei von Benzoesäure, p-Hydroxybenzoesäure und/oder oder deren Derivate oder Salze. Bevorzugt umfassen die erfindungsgemäßen Zubereitungen weniger als 0,3 Gew.% bzw. insbesondere sind sie frei von Parabenen, insbesondere frei von Methylparaben, Ethylparaben, Propylparaben, Isopropylparaben, Butylparaben, Isobutylparaben und/oder Benzylparaben. Auch 2,4-Dichlorobenzylalkohol, 4-Chloro-3,5-dimethyl-phenol, 2-Bromo-2-nitropropane-1,3-diol und/oder lodopropinylbutylcarbamate (IPBC) sowie Quatemium-15 (CAS 51229-78-8), Methyl-chloroisothiazolinone und/oder Methylisothiazolinon sind in den erfindungsgemäßen Zubereitungen nicht bzw. zu weniger als 0,3 Gew.% zu finden.
Die erfindungsgemäßen Zubereitungen sind besonders bevorzugt Parabenfrei.
Es bedeutet erfindungsgemäß "frei von" ein Anteil von weniger als 0,1 Gew.%, bezogen auf die Gesamtmasse der Zubereitung. Bevorzugt beträgt der Anteil an den zuvor genannten Mitteln und insbesondere Parabenen 0 Gew.%.

Mücken, Zecken und andere Insekten im Sinne der vorliegenden Offenbarung werden in erster Linie durch eine komplexe Duftmischung aus Milchsäure, Fettsäuren und Aminosäuren angelockt, die von der menschlichen Haut verströmt werden und den Menschen wie eine Duftglocke umgeben. Es ist nun ein Nachteil an Insektenabwehrmitteln des Standes der Technik, dass derartige Zubereitungen die natürliche, menschliche Duftkomposition nicht direkt maskieren, sondern ihr vielmehr eine zusätzliche, auf Insekten abstoßend wirkende Duftnote (die des Repellent) hinzufügen. Um das anziehende und abstoßende Reizpotential auf die Insekten über einen längeren Zeitraum zugunsten der abstoßenden Reizwirkung zu verschieben, ist es nach dem Stande der Technik notwendig, relativ große Mengen an Repellent-Wirkstoffen einzusetzen.

Die Repellent-Wirkstoffe haben einen auch für die menschliche Nase wahrnehmbaren und auf Mitmenschen nicht gerade anziehend wirkenden Eigengeruch, der die Anwendung von Insektenabwehrmitteln nicht gerade attraktiv macht.

Vorteilhaft werden der erfindungsgemäßen Zubereitung ein oder mehrere Parfuminhalststoffe zugesetzt, gewählt aus der Gruppe

- 1,3,4,6,7,8-Hexahydro-4,6,6,7,8,8-hexamethylcyclopenta-gamma-2-benzopyran
- 2,6-Dimethyl-7-octen-2-ol
- 2-Acetonapthone-1,2,3,4.5,6.7.8-octahydro-2,3,8,8-tetramethyl
- 2-Isobutyl-4-hydroxy-4-methyltetrahydropyran
- 2-tert-Pentylcyclohexylacetat
- 3,7-Dimethyl-2,6-octadien-1-ol
- 3-Methyl-5-phenyl-1-pentanol
- 7-Acetyl-1,1,3,4,4,6-hexamethyltetralin
- Adipinsäurediester
- alpha-Amylcinnamaldehyd
- alpha-Isomethytionon
- Alpha-Methylionon
- Amyl C Butylphenylmethylpropionalcinnamal
- Amylcinnamylalkohol
- Amylsalicylat
- Anisalkohol
- Benzoin
- Benzylacetat
- Benzylalkohol
- Benzylbenzoat
- Benzylcinnamat
- Benzylsalicylat
- Bergamotöl
- bitteres Orangenöl
- Butylphenylmethylpropioal
- Cardamomöl
- Cedrol
- Cinnamal
- Cinnamylalkohol
- Citral
- Citronellol
- Citronellylmethylcrotonat
- Citronenöl

- Coumarin
- Diethylsuccinat
- d-Limonene
- Ethylenbrassylate
- Ethyllinalool
- Eugenol
- Evemia Furfuracea Extract
- Evemia Prunastri Extract
- Farnesol
- Geraniol
- Guajakholzöl
- Heliotropin
- Hexylcinnamal
- Hexylsalicylat
- Hydroxycitronellal
- Hydroxyisohexyl 3-Cyclohexencarboxaldehyde
- Isoeugenol
- Lavendelöl
- Lemonenöl
- Limonen
- Linalool
- Linalylacetat
- Mandarinenöl
- Menthyl PCA
- Methyl-2 Octynoat
- Methylbenzoat
- Methylcedrylketone
- Methyldihydrojasmonate
- Methylheptenon
- Muskatnussöl
- p-t-Butyl-alpha-methyldihydrocinnamic aldehyde
- Rosmarinöl
- süßes Orangenöl
- Terpineol
- Tonkabohnenöl
- Triethylcitrat und/oder
- Vanillin.

Vorteilhaft ist der Zusatz von Hexylsalicylat, Linalylacetat und/oder 2-Isobutyl-4-hydroxy-4-methyltetrahydropyran, die den Eigengeruch des Dihydronepetalactons besonders gut maskieren ohne einen anlockenden Effekt auf die Insekten auszuüben.

Die erfindungsgemäßen Parfuminhaltstoffe, ein oder mehrere, sind bevorzugt zu einer Gesamtkonzentration von 1 ppm bis 2,5 Gew.%, jeweils bezogen auf das Gesamtgewicht der Zubereitung, in dieser enthalten.

Eine vorteilhafte Ausführungsform im Sinne der vorliegenden Erfindung ist eine kosmetische Zubereitung als Hydrodispersionsgel enthaltend 15 Gew.% IR 3535, 0,004 Gew.% Hexylsalicylat und Natriumstearoylglutamat, Sucrosepolytsearat und hydriertes Polyisobutylen, jeweils bezogen auf das Gesamtgewicht der Zubereitung.

Die Zubereitung ist bevorzugt eine kosmetische Zubereitung und zum Auftragen auf die Haut geeignet.

Die Zubereitung kann erfindungsgemäß auch in Form einer dünnflüssigen, sprühfähigen Lösung, in Form eines Gels, als Salbe, Creme oder Lotion (ggf. sprühbar) vorliegen.

Die Zubereitung kann erfindungsgemäß vorteilhaft auch als Spray oder als Tränkungsmedium für ein Pflaster oder Tuch eingesetzt werden. Daher sind auch Pflaster und Tücher getränkt mit der erfindungsgemäßen Zubereitung, erfindungsgemäß.

Zubereitungen mit Repellentien als Wirkstoff können in Form von Emulsionen und Dispersionen hergestellt werden, bei denen der lipophile Wirkstoff von einer wässrigen Phase umgeben ist (z.B. O/W-Emulsion). Durch die Umhüllung des Wirkstoffs mit der wässrigen Phase wird dessen Freisetzung aber gebremst und die Zubereitung selbst ist bei der Anwendung auf der Haut-länger wirksam gegen Mücken, Insekten, etc.

Nachteilig am Stande der Technik ist jedoch der Sachverhalt, das diese Zubereitungen aufgrund ihrer wässrigen äußeren Phase nicht besonders wasserfest sind und leicht von der Haut abgespült werden können. Ferner lassen sich

in diesen Zubereitungen keine oxidationsempfindlichen, wasserlöslichen Zusatzstoffe einarbeiten.

Diese Mängel lassen sich erfindungsgemäß durch Emulsionen mit einer wässrigen inneren Phase und einer lipophilen äußeren Phase beheben.

Die erfindungsgemäßen Zubereitung liegen daher in Form einer W/O- oder W/S-Emulsion vor.

Die erfindungsgemäßen Zubereitungen weisen dadurch eine verlängerte Wirkdauer und eine erhöhte Wasserfestigkeit auf der Haut auf.

Überraschend war insbesondere der Umstand, dass, obwohl der Repellent-Wirkstoff in der äußeren, lipophilen Phase angereichert ist, bei der Anwendung auf der Haut gleichmäßig über einen Zeitraum von mehreren Stunden an die Umwelt abgegeben wird und somit eine lang anhaltende Repellent-Wirkung entfaltet.

Überraschend war nicht zuletzt, dass bei den erfindungsgemäßen Zubereitungen die Klebrigkeit der Zubereitung auf der Haut im Vergleich zu Produkten des Stand der Technik (mit gleichem Repellent-Gehalt) deutlich reduziert ist.

Die Wasserphase der erfindungsgemäßen Zubereitung kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, Polymere, Schaumstabilisatoren, Elektrolyse, Selbstbräuner sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Eine vorhandene Ölphase kann alle herkömmlichen Bestandteile von in der Kosmetik eingesetzten Öl-, Fett- und Wachskomponenten enthalten.

Neben der erfindungsgemäßen Wirkstoff-Kombination kann die erfindungsgemäße Zubereitung weitere kosmetische Wirk- und Pflegestoffe enthalten, beispielsweise die nach der Kosmetikverordnung zugelassenen UV-Lichtschutzfilter, und Konservierungsmittel bzw. Konservierungshelfer. Derartige Wirkstoffe können erfindungsgemäß vorteilhaft in Konzentrationen von 0,01 bis 30 Gew-%, bezogen auf das Gesamtgewicht der Zubereitung in dieser enthalten sein.

Als weitere Pflegestoffe können insbesondere Niacinamid, Panthenol, Aloe vera, Hammamelis-Extrakt, Polidocanol, Vitamin E, Vitamin E-Derivate, Vitamin A, Vitamin A-Derivate, Vitamin C, Vitamin C-Derivate, Coenzym Q10, Kreatin, Taurin, Alpha-Glycosylrutin in Konzentrationen von 0,1 bis 30 Gewichts-%, bezogen auf das Gesamtgewicht der Zubereitung eingesetzt werden.

Erfindungsgemäß besonders bevorzugt enthält die erfindungsgemäße Zubereitung als weitere Bestandteile Alpha-Hydroxysäuren und/oder deren Salze. Dabei werden erfindungsgemäß bevorzugt Milchsäure/Lactate oder Zitronensäure/Citrate in einer Konzentration von 0,01 bis 5 Gewichts-%, bezogen auf das Gesamtgewicht der Zubereitung eingesetzt.

Als insbesondere kosmetische Zubereitung ist die erfindungsgemäße Zubereitung als topisch anzuwendende Mittel vorteilhaft geeignet.

Die nachfolgenden Beispiele sollen die vorliegende Erfindung verdeutlichen, ohne sie einzuschränken. Die Angaben beziehen sich stets auf Gewichts-%, sofern nicht andere Angaben gemacht werden.

**O/W-Emulsion mit Repellent**

| | 1 | 2 | 3 | 4 | 5 |
|---|---|---|---|---|---|
| Glycerylsterat | 1,0 | --- | -- | 0,5 | 0,25 |
| Stearoylglutamat, Natrium-Salz | 3,0 | 0,8 | 3,0 | 10 | 1,0 |
| Sucrosepolystearat | 3,0 | 3,0 | 0,8 | 1,0 | 10 |
| Cyclomethicon | 2,5 | 1,5 | 2.8 | 5.0 | 1,75 |
| Dimethicon | 0,5 | 1,3 | 0,5 | 1,2 | 1,75 |
| Ethylbutylacetylaminopropionat | 5 | 10 | 15 | 20 | 7,5 |
| Cetytstearylalkohol | --- | --- | 1 | 1 | --- |
| hydriertes Polyisobuten | 0,5 | 0,75 | 1,0 | 2,0 | 0,25 |
| Octyldodecanol | 0,5 | 1,0 | 0,75 | 3,0 | 0,25 |
| Parfum | q,s, | q,s, | q,s, | q,s, | q,s, |

(fortgesetzt)

| | 1 | 2 | 3 | 4 | 5 |
|---|---|---|---|---|---|
| Methylparaben | 0,4 | 0,1 | 0,05 | 0,3 | 0,4 |
| Propylparaben | 0,3 | 0,4 | 0,25 | 0,15 | --- |
| Glycerin | 5 | 10 | 3 | 15 | 7,5 |
| Ethanol | 3,0 | 1,5 | 7,5 | 1,5 | 4,5 |
| modifizierte Stärke | --- | 2,5 | --- | 0,15 | --- |
| Wasser | ad 100 | ad 100 | ad 100 | ad 100 | ad 100 |

**O/W-Emulsion mit Repellent**

| | 6 | 7 | 8 | 9 | 10 |
|---|---|---|---|---|---|
| Stearoylglutamat, Natrium-Salz | 3,0 | 0,8 | 3,0 | 10 | 1,0 |
| Sucrosepolystearat | 3,0 | 3,0 | 0,8 | 1,0 | 10 |
| Behenylalkohol | 3 | 2 | --- | 1 | --- |
| Stearylalkohol | | 2 | --- | 2 | --- |
| Cetylstearylalkohol | 3 | 4 | --- | --- | 2 |
| hydriertes Polyisobuten | 0,5 | 0,75 | 1,0 | 2,0 | 0,25 |
| Icaridin | 5 | 10 | 15 | 20 | 7,5 |
| Octyldodecanol | 0,5 | 1,0 | 0,75 | 3,0 | 0,25 |
| Glycerin | 5 | 10 | 15 | 3 | 7,5 |
| Panthenol | 0,5 | 1,0 | 0,75 | 0,25 | 0,1 |
| Parfum | q,s, | q,s, | q,s, | q,s, | q,s, |
| Methylparaben | 0,4 | 0,1 | 0,05 | 0,3 | 0,4 |
| Propylparaben | 0,3 | 0,4 | 0,25 | 0,15 | --- |
| lodopropynylbutylcarbamat | --- | --- | 0,05 | --- | 0,1 |
| Wasser | ad 100 | ad 100 | ad 100 | ad 100 | ad 100 |

**O/W-Emulsion mit Repellent**

| | 11 | 12 | 13 | 14 | 15 |
|---|---|---|---|---|---|
| Stearoylglutamat, Natrium-Salz | 3,0 | 0,8 | 3,0 | 10 | 1,0 |
| Sucrosepolystearat | 3,0 | 3,0 | 0,8 | 1,0 | 10 |
| Ethylbutylacetyleminopropionat | 5 | 5 | 15 | 20 | 7,5 |
| Dimethicon | 0,5 | 3,0 | 0,75 | 1,5 | 0,2 |
| Dicaprylylcarbonat | 3 | 5 | 10 | 15 | 5 |
| Cetylstearylalkohol | --- | --- | 1 | 1 | 0,2 |
| Tocopherol | 0,5 | 0,5 | 0,75 | 0,25 | 0,1 |
| Octyldodecanol | 0,5 | --- | 0,75 | 3,0 | 0,25 |
| Panthenol | 0,5 | --- | 0,75 | 0,25 | 0,1 |
| Carbomer | 0,05 | 0,35 | 0,15 | 0,1 | --- |

(fortgesetzt)

| | 11 | 12 | 13 | 14 | 15 |
|---|---|---|---|---|---|
| Parfum | q,s, | q,s, | q,s, | q,s, | q,s, |
| Caprylic/Capric Triglycerid | 1 | 5 | 3 | 5 | 10 |
| Methylparaben | 0,4 | 0,3 | 0,05 | 0,3 | 0,4 |
| Propylparaben | 0,3 | --- | 0,25 | 0,15 | --- |
| Benzylalkohol | --- | --- | 0,5 | --- | 1,0 |
| Phenoxyethanol | --- | 0,5 | --- | 0,15 | --- |
| Sorbitol | 10 | --- | --- | 5 | --- |
| Butylenglykol | --- | --- | --- | 5 | 10 |
| Propylenglykol | --- | --- | 10 | 5 | --- |
| Glycerin | --- | 7,5 | --- | --- | --- |
| Wasser | ad 100 | ad 100 | ad 100 | ad 100 | ad 100 |

**O/W-Emulsion mit Repellent**

| | 16 | 17 | 18 | 19 | 20 |
|---|---|---|---|---|---|
| Stearoylglutamat, Natrium-Salz | 5,0 | 0,8 | 3,0 | 10 | 1,0 |
| Sucrosepolystearat | 5,0 | 3,0 | 0,8 | 1,0 | 10 |
| Diethyltoluamid | 20 | 10 | --- | --- | 7,5 |
| Dihydronepetalactone | --- | --- | --- | 15 | --- |
| Ethylbutylacetylaminopropionat | --- | 30 | --- | --- | 7,5 |
| 3-(N-n-Butyl-N-acetyl-amino)propionsäureethylester | 5 | --- | 10 | --- | --- |
| Cyclomethicon | 2 | 4 | 6 | 1 | 3 |
| Icaridin | -- | --- | 20 | --- | --- |
| Stearylalkohol | --- | 1 | --- | 1 | 0,2 |
| Cetylstearylalkohol | --- | --- | 5,0 | 1 | 0,2 |
| Mineralöl | 0,5 | 0,75 | 1,0 | 2,0 | 0,25 |
| Octyldodecanol | 0,5 | 1,0 | 0,75 | 3.0 | 0,25 |
| Panthenol | 0,5 | 1,0 | 0,75 | 0,25 | 0,1 |
| Carbomer | 0,05 | 0,1 | 0,15 | 0,1 | --- |
| Parfum | q,s, | q,s, | q,s, | q,s, | q,s, |
| Dicaprylylcarbonat | 3 | 5 | 3,0 | 15 | 5 |
| Tocopherol | 0,5 | 1,0 | 0,75 | 0,25 | 10,1 |
| Caprylic/Capric Triglycerid | 1 | 2 | 3 | 5 | 10 |
| Methylparaben | 0,4 | 0,1 | 0,05 | 0,3 | 0,4 |
| Phenonip | 0,3 | 0,4 | 0,25 | 0,15 | --- |
| Xanthan Gum | 0,25 | 0,35 | 0,75 | --- | 1,0 |
| modifizierte Stärke | --- | 2,5 | --- | 0,15 | --- |
| Glycerin | 3 | 5 | 8 | 12 | 10 |

(fortgesetzt)

| | 16 | 17 | 18 | 19 | 20 |
|---|---|---|---|---|---|
| Wasser | ad 100 | ad 100 | ad 100 | ad 100 | ad 100 |

**Patentansprüche**

1. Zubereitung auf Emulsionsbasis umfassend

   a.) einen oder mehrere Insektenrepellentwirkstoffe und
   b.) Natriumstearoylglutamat und/oder Sucrosepolystearat sowie
   c.) hydriertes Polyisobutylen

   **dadurch gekennzeichnet, dass** die Zubereitung in Form einer W/O- oder W/S-Emulsion vorliegt.

2. Zubereitung nach Anspruch 1 **dadurch gekennzeichnet, dass** der oder die Wirkstoffe gewählt werden aus der Gruppe 1-Piperidincarboxylsäure 2-(2-hydroxyethyl)-1-methylpropylester, 3-(N-n-Butyl-N-acetyl-amino)propionsäureethylester, N,N-Diethyl-3-methylbenzamid, Dihydronepetalactone und/oder Extrakte der Katzenminze.

3. Zubereitung nach Anspruch 1 oder 2 **dadurch gekennzeichnet, dass** der Anteil an Repellentien 0,1 bis 40 Gew.% umfasst, bezogen auf die Gesamtmasse der Zubereitung.

4. Zubereitung nach einem der vorstehenden Ansprüche **dadurch gekennzeichnet, dass** als Repellent 3-(N-n-Butyl-N-acetyl-amino)propionsäureethylester gewählt wird.

5. Zubereitung nach einem der vorstehenden Ansprüche **dadurch gekennzeichnet, dass** Natriumstearoylglutamat, Sucrosepolystearat und hydriertes Polyisobutylen enthalten sind.

6. Zubereitung nach einem der vorstehenden Ansprüche **dadurch gekennzeichnet, dass** Natriumstearoylglutamat und Sucrosepolystearat im Verhältnis 1:10 bis 10:1, insbesondere 1:5 bis 5:1 eingesetzt werden.

7. Zubereitung nach einem der vorstehenden Ansprüche **dadurch gekennzeichnet, dass** die Zubereitung ein oder mehrere Parfuminhaltsstoffe gewählt aus der Gruppe 1,3,4,6,7,8-Hexahydro-4,6,6,7,8,8-hexamethylcyclopenta-gamma-2-benzopyran, 2,6-Dimethyl-7-octen-2-ol, 2-Acetonapthone-1,2,3,4,5,6,7,8-octahydro-2,3,8,8-tetramethyl, 2-Isobutyl-4-hydroxy-4-methyltetrahydropyran, 2-tert-Pentylcyclohexylacetat, 3,7-Dimethyl-2,6-octadien-1-ol, 3-Methyl-5-phenyl-1-pentanol, 7-Acetyl-1,1,3,4,4,6-hexamethyltetralin, Adipinsäurediester, alpha-Amylcinnamaldehyd, alpha-Isomethylionon, Alpha-Methylionon, Amyl C Butylphenylmethylpropionalcinnamal, Amylcinnamylalkohol, Amylsalicylat, Anisalkohol, Benzoin, Benzylacetat, Benzylalkohol, Benzylbenzoat, Benzylcinnamat, Benzylsalicylat, Bergamotöl, bitteres Orangenöl, Butylphenylmethylpropioal, Cardamomöl, Cedrol, Cinnamal, Cinnamylalkohol, Citral, Citronellol, Citronellylmethylcrotonat, Citronenöl, Coumarin, Diethylsuccinat, d-Limonene, Ethylenbrassylate, Ethyllinalool, Eugenol, Evernia Furfuracea Extract, Evernia Prunastri Extract, Farnesol, Geraniol, Guajakholzöl, Heliotropin, Hexylcinnamal, Hexylsalicylat, Hydroxycitronellal, Hydroxyisohexyl 3-Cyclohexencarboxaldehyde, Isoeugenol, Lavendelöl, Lemonenöl, Limonen, Linalool, Linalylacetat, Mandarinenöl, Menthyl PCA, Methyl-2 Octynoat, Methylbenzoat, Methylcedrylketone, Methyldihydrojasmonate, Methylheptenon, Muskatnussöl, p-t-Butyl-alpha-methyldihydrocinnamic aldehyde, Rosmarinöl, süßes Orangenöl, Terpineol, Tonkabohnenöl, Triethylcitrat und/oder Vanillin umfasst.

8. Zubereitung nach einem der vorstehenden Ansprüche **dadurch gekennzeichnet, dass** die Zubereitung ein oder mehrere Parfuminhaltsstoffe gewählt aus der Gruppe Hexylsalicylat, Linalylacetat und/oder 2-Isobutyl-4-hydroxy-4-methyltetrahydropyran umfasst.

9. Zubereitung nach einem der vorstehenden Ansprüche **dadurch gekennzeichnet, dass** die Zubereitung maximal 0,3 Gew.% bakterizide-, fungizide-, sporidzid wirksame Mittel, Konservierungsmittel und/oder Formaldehyd und Formaldehyddonatoren enthält.

10. Zubereitung nach einem der vorstehenden Ansprüche **dadurch gekennzeichnet, dass** die Zubereitung frei von

bakteriziden-, fungiziden-, sporidizid wirksamen Mitteln, Konservierungsmitteln und/oder Formaldehyd und Formaldehyddonatoren ist.

**11.** Zubereitung nach einem der vorstehenden Ansprüche **dadurch gekennzeichnet, dass** die Zubereitung frei von Parabenen ist.

**12.** Nicht-therapeutische Verwendung einer Zubereitung nach einem der Ansprüche als Insektenabwehrmittel.

**13.** Nicht-therapeutische Verwendung nach dem Anspruch 12, **dadurch gekennzeichnet, dass** die Zubereitung als Tränkungsmedium auf Vlies, Pflaster oder Tuch aufgetragen wird.

**14.** Nicht-therapeutische Verwendung der Kombination aus einem oder mehrere Insektenrepellentwirkstoffe, insbesondere 3-(N-n-Butyl-N-acetyl-amino)propionsäureethylester, und Natriumstearoylglutamat und/oder Sucrosepolytsearat sowie hydriertes Polyisobutylen zur Steigerung der Insektenabwehrwirkung um bis zu 30% gegenüber einer Zubereitung ohne Natriumstearoylglutamat und/oder Sucrosepolystearat sowie ggf. hydriertes Polyisobutylen.

## Claims

**1.** Preparation based on emulsion comprising

a.) one or more insect repellent active ingredients and
b.) sodium stearoyl glutamate and/or sucrose polystearate and
c.) hydrogenated polyisobutylene

**characterized in that** the preparation is in the form of a W/O or W/S emulsion.

**2.** Preparation according to Claim 1, **characterized in that** the active ingredient(s) is/are selected from the group of 1-methylpropyl 2-(2-hydroxyethyl)-1-piperidinecarboxylate, ethyl 3-(N-n-butyl-N-acetylamino)propionate, N,N-diethyl-3-methylbenzamide, dihydronepetalactone and/or extracts of cat.mint.

**3.** Preparation according to Claim 1 or 2, **characterized in that** the proportion of repellents comprises 0.1 to 40% by weight, based on the total mass of the preparation.

**4.** Preparation according to any of the preceding claims, **characterized in that** the repellent selected is ethyl 3-(N-n-butyl-N-acetylamino)propionate.

**5.** Preparation according to any of the preceding claims, **characterized in that** sodium stearoyl glutamate, sucrose polystearate and hydrogenated polyisobutylene are present.

**6.** Preparation according to any of the preceding claims, **characterized in that** sodium stearoyl glutamate and sucrose polystearate are used in a ratio from 1:10 to 10:1, especially from 1:5 to 5:1.

**7.** Preparation according to any of the preceding claims, **characterized in that** the preparation comprises one or more perfume ingredients selected from the group of 1,3,4,6,7,8-hexahydro-4,6,6,7,8,8-hexamethylcyclopenta-gamma-2-benzopyran, 2,6-dimethyl-7-octen-2-ol, 2-acetonaphthone-1,2,3,4,5,6,7,8-octahydro-2,3,8,8-tetramethyl, 2-isobutyl-4-hydroxy-4-methyltetrahydropyran, 2-tert-pentylcyclohexyl acetate, 3,7-dimethyl-2,6-octadien-1-ol, 3-methyl-5-phenyl-l-pentanol, 7-acetyl-1,1,3,4,4,6-hexamethyltetralin, adipic acid diester, alpha-amylcinnamaldehyde, alpha-isomethyl ionone, alphamethyl ionone, amyl C, butylphenylmethylpropional cinnamal, amyl cinnamyl alcohol, amyl salicylate, anise alcohol, benzoin, benzyl acetate, benzyl alcohol, benzyl benzoate, benzyl cinnamate, benzyl salicylate, bergamot oil, bitter orange oil, butylphenyl methylpropional, cardamom oil, cedrol, cinnamal, cinnamyl alcohol, citral, citronellol, citronellylmethyl crotonate, citrus oil, coumarin, dimethyl succinate, d-limonene, ethylene brassylate, ethyl linalool, eugenol, Evernia furfuracea extract, Evernia prunastri extract, farnesol, geraniol, guaiac wood alcohol, heliotropin, hexylcinnamal, hexyl salicylate, hydroxycitronellal, hydroxyisohexyl 3-cyclohexene carboxaldehyde, isoeugenol, lavender oil, lime oil, limonene, linalool, linalyl acetate, mandarin oil, menthyl PCA, methyl 2-octynoate, methyl benzoate, methyl cedryl ketone, methyl dihydrojasmonate, methylheptenone, nutmeg oil, p-t-butyl-alpha-methyldihydrocinnamic aldehyde, rosemary oil, sweet orange oil, terpineol, tonka bean oil, triethyl citrate and/or vanillin.

8. Preparation according to any of the preceding claims, **characterized in that** the preparation comprises one or more perfume ingredients selected from the group of hexyl salicylate, linalyl acetate and/or 2-isobutyl-4-hydroxy-4-methyltetrahydropyran.

9. Preparation according to any of the preceding claims, **characterized in that** the preparation comprises a maximum of 0.3% by weight bactericidal, fungicidal or sporicidal agents, preservatives and/or formaldehyde and formaldehyde donors.

10. Preparation according to any of the preceding claims, **characterized in that** the preparation is free of bactericidal, fungicidal or sporicidal agents, preservatives and/or formaldehyde and formaldehyde donors.

11. Preparation according to any of the preceding claims, **characterized in that** the preparation is free of parabens.

12. Non-therapeutic use of a preparation according to any of the claims as insect repellent.

13. Non-therapeutic use according to Claim 12, **characterized in that** the preparation is applied as an impregnating medium to non-wovens, plasters or tissues.

14. Non-therapeutic use of the combination of one or more insect repellent active ingredients, particularly ethyl 3-(N-n-butyl-N-acetylamino)propionate, and sodium stearoyl glutamate and/or sucrose polystearate and also hydrogenated polyisobutylene, to increase the insect repellent effect by up to 30% compared to a preparation without sodium stearoyl glutamate and/or sucrose polystearate and optionally hydrogenated polyisobutylene.

## Revendications

1. Préparation à base d'une émulsion comprenant

   a.) un ou plusieurs agents actifs répulsifs contre les insectes et
   b.) du - glutamate de stéaroyle sodique et/ou du polystéarate de sucrose, ainsi que
   c.) du polyisobutylène hydrogéné,

   **caractérisée en ce que** la réparation se présente sous la forme d'une émulsion E/H ou E/S.

2. Préparation selon la revendication 1, **caractérisée en ce que** le ou les agents actifs sont choisis dans le groupe constitué par l'ester 2-(2-hydroxyéthyl)-1-méthylpropylique de l'acide 1-pipéridine-carboxylique, l'ester éthylique de l'acide 3-(N-n-butyl-N-acétyl-amino)propionique, le N,N-diéthyl-3-méthylbenzamide, la dihydronépétalactone et/ou les extraits d'herbe à chats.

3. Préparation selon la revendication 1 ou 2, **caractérisée en ce que** la proportion de répulsifs comprend 0,1 à 40 % en poids, par rapport à la masse totale de la préparation.

4. Préparation selon l'une quelconque des revendications précédentes, **caractérisée en ce que** l'ester éthylique de l'acide 3-(N-n-butyl-N-acétyl-amino)propionique est choisi en tant que répulsif.

5. Préparation selon l'une quelconque des revendications précédentes, **caractérisée en ce que** du glutamate de stéaroyle sodique, du polystéarate de sucrose et du polyisobutylène hydrogéné sont contenus.

6. Préparation selon l'une quelconque des revendications précédentes, **caractérisée en ce que** le glutamate de stéaroyle sodique et le polystéarate de sucrose sont utilisés en un rapport de 1:10 à 10:1, notamment de 1:5 à 5:1.

7. Préparation selon l'une quelconque des revendications précédentes, **caractérisée en ce que** la préparation comprend un ou plusieurs ingrédients parfumés choisis dans le groupe constitué par le 1,3,4,6,7,8-hexahydro-4,6,6,7,8,8-hexaméthylcyclopenta-gamma-2-benzopyrane, le 2,6-diméthyl-7-octén-2-ol, le 2-acétonaphtone-1,2,3,4,5,6,7,8-octahydro-2,3,8,8-tétraméthyle, le 2-isobutyl-4-hydroxy-4-méthyltétrahydropyrane, l'acétate de 2-tert-pentylcyclohexyle, le 3,7-diméthyl-2,6-octadién-1-ol, le 3-méthyl-5-phényl-1-pentanol, la 7-acétyl-1,1,3,4,4,6-hexaméthyltétraline, les diesters de l'acide adipique, l'alpha-amylcinnamaldéhyde, l'alpha-isométhylionone, l'alpha-méthylionone, l'amyl-C-butylphénylméthylpropionalcinnamal, l'alcool amylcinnamylique, le salicylate d'amyle, l'alcool anisylique,

la benzoïne, l'acétate de benzyle, l'alcool benzylique, le benzoate de benzyle, le cinnamate de benzyle, le salicylate de benzyle, l'huilé de bergamote, l'huile d'orange amère, le butylphénylméthylpropional, l'huile de cardamome, le cédrol, le cinnamal, l'alcool cinnamylique, le citral, le citronellol, le crotonate de citronellylméthyle, l'huile de citron, la coumarine, le succinate de diéthyle, le d-limonène, le brassylate d'éthylène, l'éthyl-linalol, l'eugénol, l'extrait d'Evernia Furfuracea, l'extrait d'Evernia Prunastri, le farnésol, le géraniol, l'huile de bois de gaïac, l'héliotropine, l'hexylcinnamal, le salicylate d'hexyle, l'hydroxycitronellal, l'hydroxyisohexyl-3-cyclohexène-carboxaldéhyde, l'isoeugénol, l'huile de lavande, l'huile de citron vert, le limonène, le linalol, l'acétate de linalyle, l'huile de mandarine, le menthyl-PCA, le 2-octynoate de méthyle, le benzoate de méthyle, la méthylcédrylcétone, le dihydrojasmonate de méthyle, la méthylhepténone, l'huile de noix de muscade, l'aldéhyde p-t-butyl-alpha-méthyldihydrocinnamique, l'huile de romarin, l'huile d'orange douce, le terpinéol, l'huile de fèves tonka, le citrate de triéthyle et/ou la vanilline.

**8.** Préparation selon l'une quelconque des revendications précédentes, **caractérisée en ce que** la préparation comprend un ou plusieurs ingrédients parfumés choisis dans le groupe constitué par le salicylate d'hexyle, l'acétate de linalyle et/ou le 2-isobutyl-4-hydroxy-4-méthyltétrahydropyrane.

**9.** Préparation selon l'une quelconque des revendications précédentes, **caractérisée en ce que** la préparation contient au plus 0,3 % en poids d'agents actifs bactéricides, fongicides, sporicides, de conservateurs et/ou de formaldéhyde et de donneurs de formaldéhyde.

**10.** Préparation selon l'une quelconque des revendications précédentes, **caractérisée en ce que** la préparation est exempte d'agents actifs bactéricides, fongicides, sporicides, de conservateurs et/ou de formaldéhyde et de donneurs de formaldéhyde.

**11.** Préparation selon l'une quelconque des revendications précédentes, **caractérisée en ce que** la préparation est exempte de parabènes.

**12.** Utilisation non thérapeutique d'une préparation selon l'une quelconque des revendications précédentes en tant que substance répulsive contre les insectes.

**13.** Utilisation non thérapeutique selon la revendication 12, **caractérisée en ce que** la préparation est appliquée en tant que milieu d'imprégnation sur un non-tissé, un pansement ou une étoffe.

**14.** Utilisation non thérapeutique de la combinaison d'un ou de plusieurs agents actifs répulsifs contre les insectes, notamment d'ester éthylique de l'acide 3-(N-n-butyl-N-acétyl-amino)propionique, et de glutamate de stéaroyle sodique et/ou de polystéarate de sucrose, ainsi que de polyisobutylène hydrogéné pour augmenter l'effet répulsif contre les insectes de jusqu'à 30 % par rapport à une préparation sans glutamate de stéaroyle sodique et/ou polystéarate de sucrose, ainsi qu'éventuellement polyisobutylène hydrogéné.

Abbildung 1

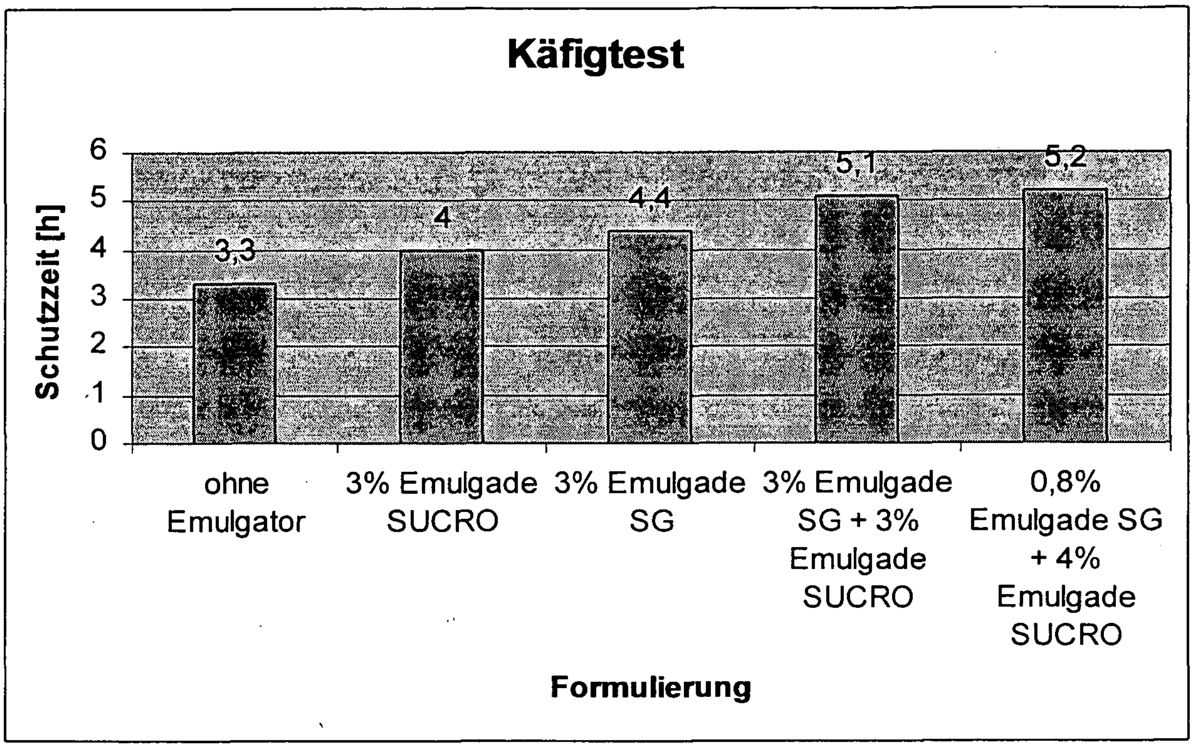
Käfigtest
Schutzzeit [h]
6
5
4
3
2
1
0
3,3
4
4,4
5,1
5,2
ohne Emulgator
3% Emulgade SUCRO
3% Emulgade SG
3% Emulgade SG + 3% Emulgade SUCRO
0,8% Emulgade SG + 4% Emulgade SUCRO
Formulierung

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 2246433
- DE 19645250
- DE 19645920
- WO 2006096876 A
- WO 2005034626 A
- DE 102005033845
- EP 1738745 A1
- EP 766959 B1
- EP 928608 B1
- EP 1151743 A1